# EUROPEAN PATENT SPECIFICATION

(11) **EP 2 137 146 B1**
(45) Date of publication and mention of the grant of the patent: **30.05.2012**
(21) Application number: 08717997.4
(22) Date of filing: 19.03.2008
(51) Int. Cl.: C07D 207/34, A61K 31/40, A61P 25/00, A61P 37/00

(54) **CRYSTALLINE FORMS OF ATORVASTATIN 4-(NITROOXY) BUTYL ESTER**
KRISTALLINE FORMEN VON ATORVASTATIN-4-(NITROOXY)BUTYLESTER
FORMES CRISTALLINES DE L'ESTER 4-(NITROOXY)BUTYLIQUE DE L'ATORVASTATINE

(30) Priority: 13.04.2007 US 907664 P
(43) Date of publication of application: 30.12.2009
(73) Proprietor: Nicox S.A., 06560 Sophia Antipolis - Valbonne (FR)
(72) Inventor: BENEDINI, Francesca, I-20097 San Donato Milanese (IT); BIONDI, Stefano, 20016 Pero (Milano) (IT); BONFANTI, Annalisa, I-20045 Besana In Brianza (IT); NICOLI, Fabio, I-20135 Milano (IT)
(74) Representative: Barchielli, Giovanna
(86) International application number: PCT/EP2008/053269
(87) International publication number: WO 2008/125412

(56) References cited:
- WO-A-2004/105754
- WO-A-2007/088123
- MOMI ET AL: "NCX 6560, a nitric oxide-releasing derivative of atorvastatin, inhibits cholesterol biosynthesis and shows anti-inflammatory and anti-thrombotic properties" EUROPEAN JOURNAL OF PHARMACOLOGY, AMSTERDAM, NL, vol. 570, no. 1-3, 16 August 2007 (2007-08-16), pages 115-124, XP022202431 ISSN: 0014-2999
- CAIRA M R: "CRYSTALLINE POLYMORPHISM OF ORGANIC COMPOUNDS" TOPICS IN CURRENT CHEMISTRY, SPRINGER, BERLIN, DE, vol. 198, 1 January 1998 (1998-01-01), pages 163-208, XP001156954 ISSN: 0340-1022

## Description

### FIELD OF THE INVENTION

The present invention relates to two crystalline forms A and B of (βR, δR) -2 (4-fluorophenyl)-β, δ-dihydroxy-5-(1-methylethyl)-3-phenyl-4-[(phenylamino)carbon-yl]-1H-pyrrole-1-heptanoic acid 4-(nitrooxy)butyl ester (atorvastatin 4-(nitrooxy) butyl ester), to methods for their preparation, to pharmaceutical compositions containing them and their use for treating and/or preventing acute coronary syndromes, stroke, neurodegenerative disorders, such as Alzheimer's and Parkinson's disease as well as autoimmune diseases, such as multiple sclerosis.

### BACKGROUND OF THE INVENTION

WO 2004/105754 discloses the process for the preparation of atorvastatin 4-(nitrooxy) butyl ester as well as its therapeutic use. Although the preparation of atorvastatin 4-(nitrooxy) butyl ester is disclosed, WO 2004/105754 is silent with respect to the crystalline form of the compound. The procedure disclosed in the examples leads to the amorphous form which has been demonstrated by powder X-ray analysis.
It has been found that the amorphous form of Atorvastatin 4-(nitrooxy) butyl ester is unstable at humidity conditions, in organic solvents and in stressed thermal conditions. Therefore the instability of the amorphous form of Atorvastatin 4-(nitrooxy) butyl ester complicates the development of solid formulations.
Therefore there is an ongoing need to find Atorvastatin 4-(nitrooxy) butyl ester forms which are stable and easy to purify for the preparation of pharmaceutical formulations. Finally, it is economically desirable that the product is stable for extended periods of time without the need for specialized storage conditions.

It has now unexpectedly been found two crystalline forms, which have been designated as form A and form B, of atorvastatin 4-(nitrooxy) butyl ester. Each of the new forms is differentiated by a unique X-ray powder diffraction pattern, and a unique Raman spectrum.
The two crystalline forms A and B of atorvastatin 4-(nitrooxy) butyl ester have good stability in water, in organic solvents, and when exposed to heat and humidity.
Moreover, the isolation of the two crystalline forms is a convenient purification procedure for the industrial scale production of (βR, δR)-2(4-fluorophenyl)-β, δ-dihydroxy-5-(1-methylethyl)-3-phenyl-4-[(phenylamino)carbon-yl]-1H-pyrrole-1-heptanoic acid 4-(nitrooxy)butyl ester in high chemical purity which is required to meet the pharmaceutical quality.

### SUMMARY OF THE INVENTION

The present invention relates to two crystalline forms A and B of (βR, δR)-2(4-fluorophenyl)-β, δ-dihydroxy-5-(1-methylethyl)-3-phenyl-4-[(phenylamino)carbon-yl]-1H-pyrrole-1-heptanoic acid 4-(nitrooxy) butyl ester (atorvastatin 4-(nitrooxy) butyl ester)of Formula (I) 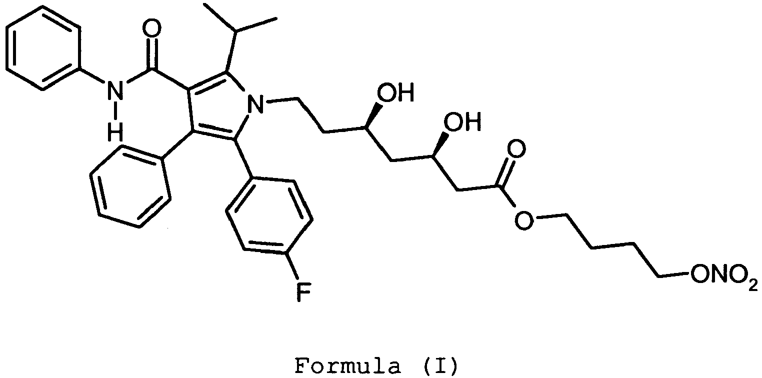 and processes for the preparation thereof.

### BRIEF DESCRIPTION OF THE DRAWINGS

FIGURE 1 is a X-ray powder diffraction pattern of form B of Atorvastatin 4-(nitrooxy) butyl ester.
FIGURE 2 is a Raman spectrum of form B of Atorvastatin 4-(nitrooxy) butyl ester.
FIGURE 3 is a differential scanning calorimetric thermogram of form B of Atorvastatin 4-(nitrooxy) butyl ester.
FIGURE 4 is a X-ray powder diffraction pattern of form A of Atorvastatin 4-(nitrooxy) butyl ester.
FIGURE 5 is a Raman spectrum of form A of Atorvastatin 4-(nitrooxy) butyl ester.
FIGURE 6 is a differential scanning calorimetric thermogram of form A of Atorvastatin 4-(nitrooxy) butyl ester.

### DETAILED DESCRIPTION OF THE INVENTION

One object of the present invention is directed to a crystalline form, herein designated as form B, of (βR, δR)-2(4-fluorophenyl)-β, δ-dihydroxy-5-(1-methylethyl)-3-phenyl-4-[(phenylamino)carbon-yl]-1H-pyrrole-1-heptanoic acid 4-(nitrooxy) butyl ester (atorvastatin 4-(nitrooxy) butyl ester), which has the X-ray powder diffraction (PXRD) pattern having peaks of intensity percentage higher than 10%, at 2-theta (2θ) = 9.47±0.1; 14.26±0.1; 15.03±0.1; 16.97±0.1; 18.70+0.1; 19.04±0.1; 19.71±0.1; 19.92±0.1; 20.82+0.1; 21.21±0.1; 21.77±0.1; 22.17±0.1; 22.44±0.1; 22.67+0.1; 23.97±0.1; 24.89±0.1; 25.24±0.1; 28.23±0.1; 30.36±0.1; 33.54±0.1 as depicted in Table 1;

**TABLE 1 X-ray powder diffraction values form B**

| Angle [2-Theta °] | d value [Angstrom] | Intensity [Cps] | Intensity % [%] |
|---|---|---|---|
| 7.18 | 12.3 | 7.38 | 5.1 |
| 9.47 | 9.3 | 73.7 | 51.4 |
| 10.82 | 8.2 | 4.6 | 3.2 |
| 11.95 | 7.4 | 5.39 | 3.8 |
| 14.26 | 6.2 | 18.9 | 13.2 |
| 14.47 | 6.1 | 8.12 | 5.7 |
| 15.03 | 5.89 | 17.9 | 12.5 |
| 15.58 | 5.68 | 10.3 | 7.2 |
| 16.29 | 5.44 | 8.49 | 5.9 |
| 16.66 | 5.32 | 10.1 | 7.1 |
| 16.97 | 5.22 | 31.6 | 22 |
| 17.86 | 4.96 | 12.7 | 8.9 |
| 18.13 | 4.89 | 10.9 | 7.6 |
| 18.70 | 4.74 | 144 | 100 |
| 19.04 | 4.66 | 33.2 | 23.1 |
| 19.71 | 4.50 | 43 | 29.9 |
| 19.92 | 4.45 | 64.8 | 45.1 |
| 20.49 | 4.33 | 8 | 5.6 |
| 20.82 | 4.26 | 27.2 | 18.9 |
| 21.21 | 4.19 | 26.9 | 18.7 |
| 21.77 | 4.08 | 31.3 | 21.8 |
| 22.17 | 4.01 | 42.4 | 29.5 |
| 22.44 | 3.96 | 23 | 16 |
| 22.67 | 3.92 | 42.7 | 29.7 |
| 23.32 | 3.81 | 13.7 | 9.6 |
| 23.62 | 3.76 | 8.33 | 5.8 |
| 23.97 | 3.71 | 31.7 | 22.1 |
| 24.56 | 3.62 | 11.5 | 8 |
| 24.89 | 3.57 | 37.7 | 26.3 |
| 25.24 | 3.53 | 16.1 | 11.2 |
| 25.69 | 3.46 | 11 | 7.7 |
| 25.97 | 3.43 | 8.05 | 5.6 |
| 26.72 | 3.33 | 10.2 | 7.1 |
| 27.06 | 3.29 | 13.2 | 9.2 |
| 27.50 | 3.24 | 5.69 | 4 |
| 28.23 | 3.16 | 20 | 13.9 |
| 28.66 | 3.11 | 8.64 | 6 |
| 29.16 | 3.06 | 8.46 | 5.9 |
| 29.41 | 3.03 | 13.9 | 9.7 |
| 30.36 | 2.94 | 14.9 | 10.4 |
| 30.61 | 2.92 | 7.88 | 5.5 |
| 30.97 | 2.88 | 5.45 | 3.8 |
| 31.26 | 2.86 | 9.22 | 6.4 |
| 31.49 | 2.84 | 7.09 | 4.9 |
| 32.28 | 2.77 | 9.78 | 6.8 |
| 32.85 | 2.72 | 7.28 | 5.1 |
| 33.13 | 2.70 | 7.65 | 5.3 |
| 33.54 | 2.67 | 15.9 | 11.1 |
| 33.85 | 2.65 | 8.7 | 6.1 |
| 34.22 | 2.62 | 8.16 | 5.7 |
| 34.44 | 2.60 | 12.1 | 8.4 |
| 35.25 | 2.54 | 8.15 | 5.7 |
| 35.98 | 2.49 | 8.17 | 5.7 |
| 36.51 | 2.46 | 5.62 | 3.9 |
| 37.07 | 2.42 | 6.96 | 4.8 |
| 38.02 | 2.36 | 11.5 | 8 |
| 38.36 | 2.34 | 6.54 | 4.6 |
| 38.60 | 2.33 | 5.45 | 3.8 |

Form B of atorvastatin 4-(nitrooxy) butyl ester is further characterized by Raman spectroscopy, having the main absorption peaks at wavelength (λ) cm⁻¹: 3064; 2963; 2947; 2943; 2918; 2890; 1665; 1603; 1560; 1528; 1508; 1481; 1452; 1435; 1409; 1400; 1365; 1311; 1241; 1182; 1159; 1036; 1006; 996; 825; 198; 112; 86 as depicted in Table 2;

**TABLE 2: Raman spectroscopy absorption peaks of Form B**

| λ | absolute | intensity | | λ | absolute | intensity |
|---|---|---|---|---|---|---|
| [cm⁻¹] | intensity | (%) | | [cm⁻¹] | intensity | (%) |
| 3064 | 0.105 | 34.9 | | 1073 | 0.019 | 6.3 |
| 2995 | 0.019 | 6.3 | | 1036 | 0.03 | 10.0 |
| 2963 | 0.066 | 21.9 | | 1006 | 0.047 | 15.6 |
| 2947 | 0.064 | 21.3 | | 996 | 0.071 | 23.6 |
| 2943 | 0.064 | 21.3 | | 964 | 0.008 | 2.7 |
| 2918 | 0.065 | 21.6 | | 939 | 0.006 | 2.0 |
| 2890 | 0.046 | 15.3 | | 898 | 0.016 | 5.3 |
| 2858 | 0.017 | 5.6 | | 871 | 0.021 | 7.0 |
| 2560 | 0.005 | 1.7 | | 856 | 0.026 | 8.6 |
| 1730 | 0.011 | 3.7 | | 825 | 0.046 | 15.3 |
| 1665 | 0.09 | 29.9 | | 811 | 0.011 | 3.7 |
| 1603 | 0.208 | 69.1 | | 790 | 0.005 | 1.7 |
| 1579 | 0.02 | 6.6 | | 767 | 0.013 | 4.3 |
| 1560 | 0.035 | 11.6 | | 732 | 0.011 | 3.7 |
| 1528 | 0.088 | 29.2 | | 712 | 0.008 | 2.7 |
| 1508 | 0.038 | 12.6 | | 685 | 0.009 | 3.0 |
| 1481 | 0.046 | 15.3 | | 661 | 0.008 | 2.7 |
| 1467 | 0.026 | 8.6 | | 632 | 0.017 | 5.6 |
| 1452 | 0.031 | 10.3 | | 617 | 0.019 | 6.3 |
| 1435 | 0.035 | 11.6 | | 585 | 0.009 | 3.0 |
| 1409 | 0.045 | 15.0 | | 566 | 0.009 | 3.0 |
| 1400 | 0.054 | 17.9 | | 517 | 0.014 | 4.7 |
| 1381 | 0.021 | 7.0 | | 473 | 0.006 | 2.0 |
| 1365 | 0.034 | 11.3 | | 433 | 0.006 | 2.0 |
| 1337 | 0.012 | 4.0 | | 417 | 0.011 | 3.7 |
| 1311 | 0.033 | 11.0 | | 406 | 0.008 | 2.7 |
| 1298 | 0.024 | 8.0 | | 377 | 0.01 | 3.3 |
| 1283 | 0.02 | 6.6 | | 364 | 0.014 | 4.7 |
| 1265 | 0.014 | 4.7 | | 350 | 0.018 | 6.0 |
| 1241 | 0.052 | 17.3 | | 298 | 0.008 | 2.7 |
| 1228 | 0.022 | 7.3 | | 268 | 0.012 | 4.0 |
| 1182 | 0.037 | 12.3 | | 248 | 0.028 | 9.3 |
| 1159 | 0.05 | 16.6 | | 198 | 0.053 | 17.6 |
| 1120 | 0.012 | 4.0 | | 112 | 0.301 | 100.0 |
| 1108 | 0.016 | 5.3 | | 86 | 0.179 | 59.5 |

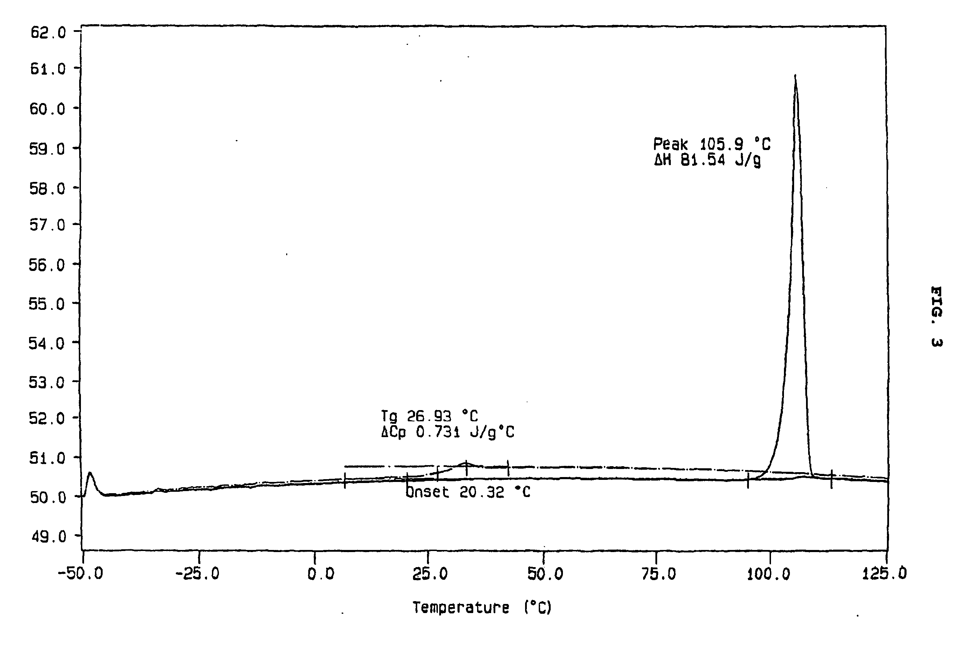

Form B of atorvastatin 4-(nitrooxy) butyl ester is also characterized by Differential Scanning Calorimetry (DSC) performed using a heating rate of 10 K min⁻¹ in closed gold crucibles, showing a melting endotherm with a peak maximum at 104- 105°C as given in Figure 3.

Another object of the present invention relates to the crystalline form, herein designated as form A, of (βR, δR)-2(4-fluorophenyl)-β, δ-dihydroxy-5-(1-methylethyl)-3-phenyl-4-[(phenylamino)carbon-yl]-1H-pyrrole-1-heptanoic acid 4-(nitrooxy) butyl ester (atorvastatin 4-(nitrooxy) butyl ester), which has the X-ray powder diffraction (PXRD) pattern having peaks of intensity percentage higher than 10%, at 2-theta (2θ) = 9.19±0.1; 10.42±0.1; 11.49±0.1; 18.48±0.1; 18.98±0.1; 19.53±0.1; 19.68±0.1; 20.22±0.1; 20.80+0.1; 21.04±0.1; 21.52±0.1; 21.77±0.1; 23.16±0.1; 23.53±0.1; 25.66±0.1; 26.78±0.1; 27.85±0.1 as depicted in Table 3;

**TABLE 3: X-ray powder diffraction values form A**

| Angle | d value | Intensity | Intensity % |
|---|---|---|---|
| [2-Theta °] | [Angstrom] | [Cps] | [%] |
| 5.18 | 17.0 | 7.88 | 4.6 |
| 5.48 | 16.1 | 4.82 | 2.8 |
| 7.09 | 12.5 | 6.61 | 3.9 |
| 9.19 | 9.6 | 33.2 | 19.3 |
| 9.71 | 9.1 | 5.94 | 3.5 |
| 10.42 | 8.5 | 172 | 100 |
| 11.49 | 7.7 | 29.5 | 17.2 |
| 14.27 | 6.2 | 5.26 | 3.1 |
| 14.83 | 5.97 | 9.69 | 5.7 |
| 15.42 | 5.74 | 12 | 7 |
| 16.14 | 5.49 | 11.4 | 6.6 |
| 16.58 | 5.34 | 8.38 | 4.9 |
| 17.79 | 4.98 | 9.31 | 5.4 |
| 17.97 | 4.93 | 12.1 | 7 |
| 18.48 | 4.80 | 103 | 60.3 |
| 18.98 | 4.67 | 43.4 | 25.3 |
| 19.53 | 4.54 | 17.7 | 10.3 |
| 19.68 | 4.51 | 23.4 | 13.7 |
| 20.22 | 4.39 | 18.8 | 11 |
| 20.80 | 4.27 | 36.6 | 21.4 |
| 21.04 | 4.22 | 22 | 12.8 |
| 21.52 | 4.13 | 65 | 37.9 |
| 21.77 | 4.08 | 18.6 | 10.8 |
| 22.55 | 3.94 | 15.7 | 9.2 |
| 23.16 | 3.84 | 57.1 | 33.3 |
| 23.53 | 3.78 | 46 | 26.8 |
| 24.03 | 3.70 | 14.3 | 8.3 |
| 24.38 | 3.65 | 12.8 | 7.4 |
| 24.96 | 3.57 | 14.7 | 8.6 |
| 25.66 | 3.47 | 45.7 | 26.6 |
| 26.45 | 3.37 | 12 | 7 |
| 26.78 | 3.33 | 20.8 | 12.1 |
| 27.85 | 3.20 | 18.4 | 10.7 |
| 28.69 | 3.11 | 6.81 | 4 |
| 29.47 | 3.03 | 16.9 | 9.8 |
| 29.93 | 2.98 | 8.66 | 5 |
| 30.24 | 2.95 | 8.68 | 5.1 |
| 30.85 | 2.90 | 7.14 | 4.2 |
| 31.71 | 2.82 | 10.9 | 6.4 |
| 32.09 | 2.79 | 11.3 | 6.6 |
| 32.93 | 2.72 | 6.85 | 4 |
| 33.23 | 2.69 | 8.24 | 4.8 |
| 34.38 | 2.61 | 10.8 | 6.3 |
| 35.06 | 2.56 | 7.86 | 4.6 |
| 36.25 | 2.48 | 13.1 | 7.6 |
| 36.73 | 2.45 | 7.62 | 4.4 |
| 37.81 | 2.38 | 6.27 | 3.7 |
| 38.33 | 2.35 | 9.18 | 5.4 |
| 39.15 | 2.30 | 6.67 | 3.9 |

Form A of atorvastatin 4-(nitrooxy) butyl ester is further characterized by Raman spectroscopy, having the main absorption peaks at wavelength (λ) cm⁻¹: 3057; 2968; 2944; 2929; 2919; 1662; 1605; 1533; 1509; 1481; 1462; 1446; 1423; 1409; 1380; 1367; 1313; 1280; 1243; 1180; 1156; 1035; 1006; 997; 880; 857; 227; 201; 101; 85 as depicted in Table 4;

**TABLE 4: Raman spectroscopy absorption peaks form A**

| λ | absolute | intensity | | λ | absolute | intensity |
|---|---|---|---|---|---|---|
| [cm⁻¹] | intensity | (%) | | [cm⁻¹] | intensity | (%) |
| 3064 | 0.054 | 28.0 | | 1054 | 0.012 | 6.2 |
| 3057 | 0.055 | 28.5 | | 1035 | 0.035 | 18.1 |
| 3007 | 0.018 | 9.3 | | 1006 | 0.041 | 21.2 |
| 2968 | 0.037 | 19.2 | | 997 | 0.068 | 35.2 |
| 2944 | 0.052 | 26.9 | | 880 | 0.021 | 10.9 |
| 2929 | 0.05 | 25.9 | | 857 | 0.027 | 14.0 |
| 2919 | 0.052 | 26.9 | | 835 | 0.013 | 6.7 |
| 2550 | 0.006 | 3.1 | | 823 | 0.018 | 9.3 |
| 1728 | 0.009 | 4.7 | | 807 | 0.012 | 6.2 |
| 1662 | 0.091 | 47.2 | | 765 | 0.009 | 4.7 |
| 1605 | 0.141 | 73.1 | | 734 | 0.013 | 6.7 |
| 1580 | 0.018 | 9.3 | | 708 | 0.007 | 3.6 |
| 1565 | 0.015 | 7.8 | | 693 | 0.008 | 4.1 |
| 1533 | 0.096 | 49.7 | | 660 | 0.006 | 3.1 |
| 1509 | 0.032 | 16.6 | | 635 | 0.015 | 7.8 |
| 1481 | 0.041 | 21.2 | | 618 | 0.017 | 8.8 |
| 1462 | 0.022 | 11.4 | | 581 | 0.014 | 7.3 |
| 1446 | 0.028 | 14.5 | | 512 | 0.015 | 7.8 |
| 1423 | 0.027 | 14.0 | | 471 | 0.006 | 3.1 |
| 1409 | 0.047 | 24.4 | | 441 | 0.006 | 3.1 |
| 1380 | 0.022 | 11.4 | | 409 | 0.009 | 4.7 |
| 1367 | 0.045 | 23.3 | | 384 | 0.01 | 5.2 |
| 1313 | 0.029 | 15.0 | | 367 | 0.013 | 6.7 |
| 1280 | 0.022 | 11.4 | | 330 | 0.013 | 6.7 |
| 1243 | 0.054 | 28.0 | | 243 | 0.017 | 8.8 |
| 1180 | 0.024 | 12.4 | | 227 | 0.026 | 13.5 |
| 1156 | 0.046 | 23.8 | | 201 | 0.027 | 14.0 |
| 1113 | 0.015 | 7.8 | | 101 | 0.193 | 100.0 |
| 1085 | 0.012 | 6.2 | | 85 | 0.166 | 86.0 |

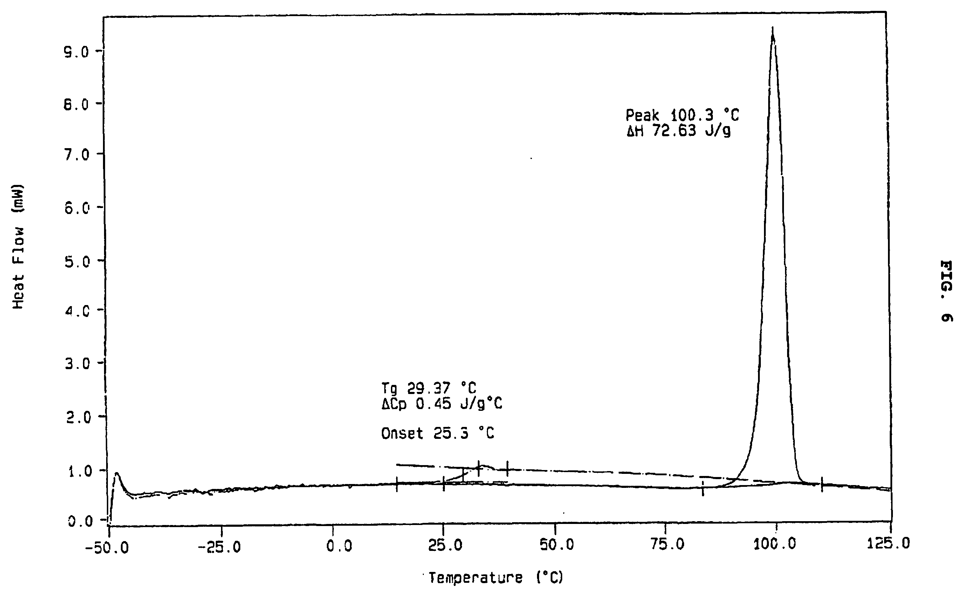

Form A of atorvastatin 4-(nitrooxy) butyl ester is further characterized by Differential Scanning Calorimetry (DSC), performed by heating at a rate of 10 K min⁻¹ in closed gold crucibles, showing a melting endotherm with a peak maximum at 98-100°C as given in Figure 6.
For the preparation of the crystalline forms, there may be used crystallisation techniques well known in the art, such as suspension, precipitation, re-crystallisation or evaporation. Diluted, saturated or super-saturated solutions may be used for crystallisation, with or without seeding with suitable nucleating agents. Temperatures up to the boiling point of the solvent (solvent mixture) may be applied to form solutions. Cooling to initiate crystallisation and precipitation down to - 5°C and preferably down to room temperature may be applied.

Atorvastatin 4-(nitrooxy) butyl ester form B is prepared by a process comprising the following steps:
stirring a suspension of amorphous atorvastatin 4-(nitrooxy) butyl ester in cumene at -5°C;
adding further cumene to complete the precipitation;
collecting the solid by filtration.

In a second procedure, atorvastatin 4-(nitrooxy) butyl ester form B is prepared by a process comprising the following steps:
stirring a suspension of amorphous atorvastatin 4-(nitrooxy) butyl ester in 1-octanol at 40°C; adding further 1-octanol to complete the precipitation;
collecting the solid by filtration.

Another process for preparing atorvastatin 4-(nitrooxy) butyl ester form B comprises the following steps:
stirring a suspension of amorphous atorvastatin 4-(nitrooxy) butyl ester in a mixture of ethyl acetate/heptane 1:2 (V/V) at 5°C;
adding further cold ethyl acetate/heptane 1:2 (V/V) to complete the precipitation;
collecting the solid by filtration.

In a further procedure, atorvastatin 4-(nitrooxy) butyl ester form B is prepared by a process comprising the following steps:
stirring a suspension of amorphous atorvastatin 4-(nitrooxy) butyl ester in a mixture of ethyl acetate/hexane 1:1 (V/V) at room temperature;
adding further hexane to complete the precipitation;
collecting the solid by filtration.

Atorvastatin 4-(nitrooxy) butyl ester form B is also prepared by a process comprising the following steps:
stirring a suspension of amorphous atorvastatin 4-(nitrooxy) butyl ester in a mixture of toluene/isopropyl ether about 1:2 (V/V) at 20-35°C;
collecting the solid by centrifugation.

Another process for preparing atorvastatin 4-(nitrooxy) butyl ester form B comprises the following steps:
dissolving amorphous atorvastatin 4-(nitrooxy) butyl ester in a mixture of ethyl acetate/hexane 0.5:1 (V/V) by heating to 50°C;
precipitating the solid by rapidly cooling the solution to 0°C;
collecting the solid by filtration.

A further process for preparing atorvastatin 4-(nitrooxy) butyl ester form B comprises the following steps:
dissolving amorphous atorvastatin 4-(nitrooxy) butyl ester in a mixture of ethyl acetate/hexane 1:1 (V/V) at room temperature;
precipitating the solid by exposing the solution to an hexane saturated atmosphere;
collecting the solid by filtration.

A still another process for preparing atorvastatin 4-(nitrooxy) butyl ester form B comprises the following steps: dissolving amorphous atorvastatin 4-(nitrooxy) butyl ester in a mixture of ethyl acetate/hexane 2:1 (V/V) at room temperature; precipitating the solid by exposing the solution to an hexane saturated atmosphere over night and collecting the solid by filtration.

Atorvastatin 4-(nitrooxy) butyl ester form A can be prepared by a process comprising the following steps: dissolving atorvastatin 4-(nitrooxy) butyl ester form B in terbutyl methyl ether at room temperature;
adding heptane to achieve the precipitation;
shaking the suspension;
further adding heptane to complete the precipitation; collecting the solid by filtration.

Another process for preparing atorvastatin 4-(nitrooxy) butyl ester form A comprises the following steps:
dissolving atorvastatin 4-(nitrooxy) butyl ester form B in a mixture of ethyl acetate/hexane 2:1 (V/V) at room temperature;
precipitating the solid by adding hexane;
shaking the suspension;
collecting the solid by filtration.

Another aspect of the present invention provides the use of atorvastatin 4-(nitrooxy) butyl ester crystalline form A or B of formula (I) as a medicament having anti-inflammatory, antithrombotic and antiplatelet activity, used to treat acute coronary syndromes, stroke, peripheral vascular diseases and all disorders associated with endothelial dysfunctions and for treating and/or preventing neurodegenerative disorders such as Alzheimer's and Parkinson's disease as well as autoimmune disorders such as multiple sclerosis.

The present invention also provides pharmaceutical compositions comprising at least atorvastatin 4-(nitrooxy) butyl ester crystalline form A or B together with non toxic adiuvants and/or carriers usually employed in the pharmaceutical field.

Another aspect of the present invention provides the pharmaceutical composition comprising atorvastatin 4-(nitrooxy) butyl ester crystalline form A or B in combination with at least a compound used to treat cardiovascular diseases selected from the group comprising: ACE inhibitors, angiotensin II receptor antagonists, beta-adrenergic blockers, calcium channel blockers, antithrombotics such as aspirin, nitrosated ACE inhibitors, nitrosated angiotensin II receptor antagonists, nitrosated beta-adrenergic blockers and nitrosated aspirin.

The daily dose of active ingredient that should be administered can be a single dose or it can be an effective amount divided into several smaller doses that are to be administered throughout the day. Usually, total daily dose may be in amounts preferably from 5 to 1000 mg. The dosage regimen and administration frequency for treating the mentioned diseases with the compound of the invention and/or with the pharmaceutical compositions of the present invention will be selected in accordance with a variety of factors, including for example age, body weight, sex and medical condition of the patient as well as severity of the disease, route of administration, pharmacological considerations and eventual concomitant therapy with other drugs. In some instances, dosage levels below or above the aforesaid range and/or more frequent may be adequate, and this logically will be within the judgment of the physician and will depend on the disease state.

The two compounds of the invention may be administered orally, parenterally, rectally or topically, by inhalation or aerosol, in formulations eventually containing conventional non-toxic pharmaceutically acceptable carriers, adjuvants and vehicles as desired. Topical administration may also involve the use of transdermal administration such as transdermal patches or iontophoresis devices. The term "parenteral" as used herein, includes subcutaneous injections, intravenous, intramuscular, intrasternal injection or infusion techniques.

Injectable preparations, for example sterile injectable aqueous or oleaginous suspensions may be formulated according to known art using suitable dispersing or wetting agents and suspending agents. The sterile injectable preparation may also be a sterile injectable solution or suspension in a non-toxic parenterally acceptable diluent or solvent. Among the acceptable vehicles and solvents are water, Ringer's solution and isotonic sodium chloride. In addition, sterile, fixed oils are conventionally employed as a solvent or suspending medium. For this purpose any bland fixed oil may be employed including synthetic mono or diglycerides, in addition fatty acids such as oleic acid find use in the preparation of injectables.

Suppositories for rectal administration of the drug can be prepared by mixing the active ingredient with a suitable nonirritating excipient, such as cocoa butter and polyethylene glycols.

Solid dosage forms for oral administration may include capsules, tablets, pills, powders, granules and gels. In such solid dosage forms, the active compound may be admixed with at least one inert diluent such as sucrose, lactose or starch. Such dosage forms may also comprise, as in normal practice, additional substances other than inert diluents, e.g. lubricating agents such as magnesium stearate. In the case of capsules, tablets and pills, the dosage forms may also comprise buffering agents. Tablets and pills can additionally be prepared with enteric coatings.

Liquid dosage forms for oral administration may include pharmaceutically acceptable emulsions, solutions, suspensions, syrups and elixirs containing inert diluents commonly used in the art, such as water. Such compositions may also comprise adjuvants, such as wetting agents, emulsifying and suspending agents, and sweetening, flavouring and the like.

### Experimental part

The crystalline forms have been characterized by X-ray powder diffraction, Raman spectroscopy, and DSC under the following experimental conditions:
X-ray powder diffraction data were acquired using a Bruker e X-ray diffractometer model D8 Advance. System description: Copper Kα radiation, voltage 35 kV, current 45 mA;
Raman Spectroscopy data acquired using a Bruker RFS100 instrument with OPUS 3.1 software; Nd:YAG 1064 nm excitation; 100 mW laser power; Ge detector; 64 scans; 3500-25 cm⁻¹ range; 2 cm⁻¹ resolution;
Differential Scanning Calorimetry experiments run on a Perkin Elmer DSC 7 or Perkin Elmer Pyris 1. The samples were analyzed inside closed aluminium or gold crucibles, filling the sample in an N₂ environment. The heating rate was 10 K min⁻¹, -50 to 125°C range.

Amorphous atorvastatin 4-(nitrooxy) butyl ester was prepared according to example 7 of WO 2004/105754.

### Example 1

### Preparation of atorvastatin 4-(nitrooxy) butyl ester form B

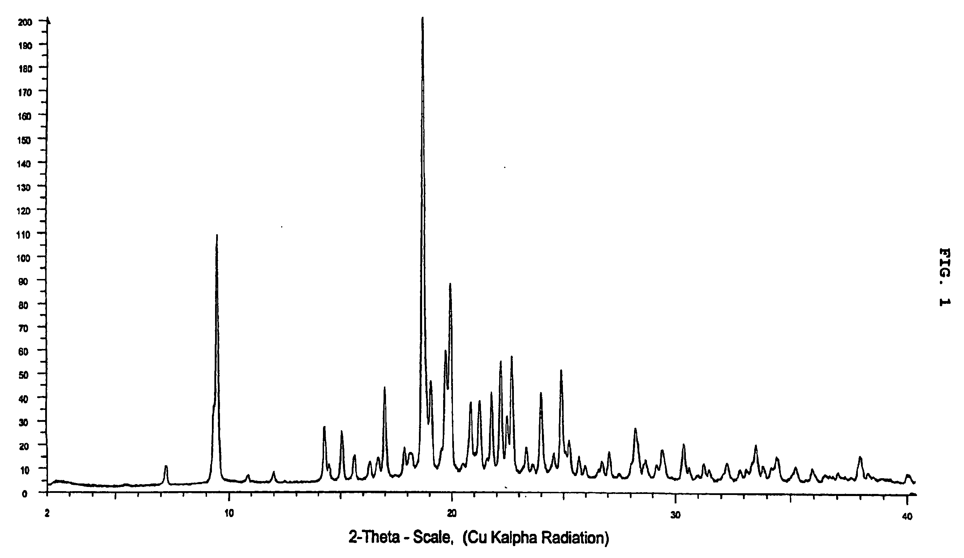

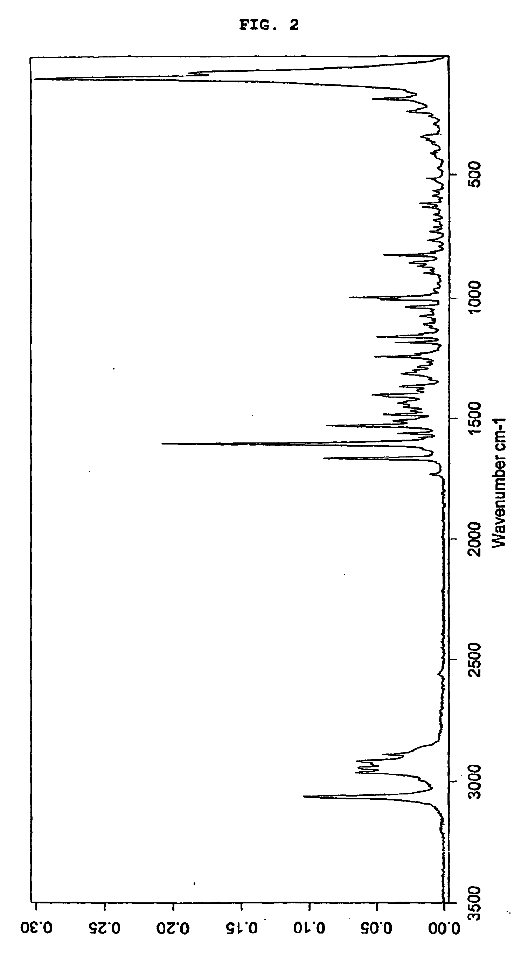

51 mg of amorphous atorvastatin 4-(nitrooxy) butyl ester were suspended in 0.2 ml of cumene. The suspension was stirred at - 5°C. After 15 minutes further 1.8 ml of cumene were added. The white solid was filtered off after total 21.5 h of stirring. The obtained product is crystal B which is characterized by an X-ray powder diffraction pattern as shown in Figure 1 and further characterized by Raman spectroscopy giving the spectrum shown in Figure 2. Differential Scanning Calorimetry showed the sample to have a melting endotherm with a peak maximum at 104-105°C as given in Figure 3.

### Example 2

### Preparation of atorvastatin 4-(nitrooxy) butyl ester form B

100.4 mg of amorphous atorvastatin 4-(nitrooxy) butyl ester were suspended in 0.2 ml of 1-octanol. The suspension was heated to 40°C under stirring. After 20 minutes further 1.8 ml of 1-octanol were added. After additional 4 hours of stirring the solid was filtered off. X-Ray powder diffraction pattern and Raman spectrum agree with that of form B given in Example 1.

### Example 3

### Preparation of atorvastatin 4-(nitrooxy) butyl ester form B

105.1 mg of atorvastatin 4-(nitrooxy) butyl ester amorphous form were stirred for 1 hour in 0.3 ml of cold ethyl acetate/heptane 1:2 (V/V) at 5°C. The precipitation was completed by the addition of further 1.7 ml of cold ethyl acetate/heptane 1:2 (V/V). The solid was collected by filtration. X-Ray powder diffraction pattern and Raman spectrum agree with that of form B given in Example 1.

### Example 4

### Preparation of atorvastatin 4-(nitrooxy) butyl ester form B

141.9 mg of atorvastatin 4-(nitrooxy) butyl ester amorphous form were suspended in 0.3 ml of cold ethyl acetate/hexane 1:1 (V/V). To the cloudy solution under stirring additional 0.5 ml of hexane were added after 10 minutes, giving a white solid that was collected by filtration. X-Ray powder diffraction pattern and Raman spectrum agree with that of form B given in Example 1.

### Example 5

### Preparation of atorvastatin 4-(nitrooxy) butyl ester form B

74.5 mg of atorvastatin 4-(nitrooxy) butyl ester form B were suspended in 3.5 ml of toluene and 6 ml of isopropyl ether. After stirring at 20-35°C for 11 days the product was recovered by centrifugation. X-Ray powder diffraction pattern and Raman spectrum agree with that of form B given in Example 1.

### Example 6

### Preparation of atorvastatin 4-(nitrooxy) butyl ester form B

73.2 mg of atorvastatin 4-(nitrooxy) butyl ester form B were dissolved in 3.5 ml of a mixture ethyl acetate/hexane 1:2(v/v) at 50°C. The clear solution was stirred for 5 minutes, than it was rapidly cooled down to 0°C and stirred for 1.5h. The suspension was then stirred at room temperature for additional 1 h and filtered under vacuum. 37.3 mg of a white powder were obtained. X-Ray powder diffraction pattern and Raman spectrum agree with that of form B given in Example 1.

### Example 7

### Preparation of atorvastatin 4-(nitrooxy) butyl ester form B

84.5 mg of atorvastatin 4-(nitrooxy) butyl ester form B were dissolved in 3.5 ml of ethyl acetate/hexane 1:1 (V/V). The solution stored into an open vial was put into a larger closed vial containing a hexane reservoir. Precipitation was observed within 4 days. The solid was recovered after filtration under vacuum leading to 68.5 mg of white solid. X-Ray powder diffraction pattern and Raman spectrum agree with that of form B given in Example 1.

### Example 8

### Preparation of atorvastatin 4-(nitrooxy) butyl ester form B

82.9 mg of atorvastatin 4-(nitrooxy) butyl ester form B were dissolved in 0.8 ml of ethyl acetate/hexane 2:1 (V/V). The solution was stored into an open vial and it was put into a larger closed vial containing a hexane reservoir. The suspension was stirred over night. The solid was recovered after filtration under vacuum leading to 68.5 mg of white solid. X-Ray powder diffraction pattern and Raman spectrum agree with that of form B given in Example 1.

### Example 9

### Preparation of atorvastatin 4-(nitrooxy) butyl ester form A

295.8 mg of atorvastatin 4-(nitrooxy) butyl ester form B were dissolved in 2.8 ml of ethyl acetate/hexane 2:1 (V/V). 7.2 ml of hexane were added. The suspension was shaken for 1.5 h at room temperature, then the solid was filtered off.

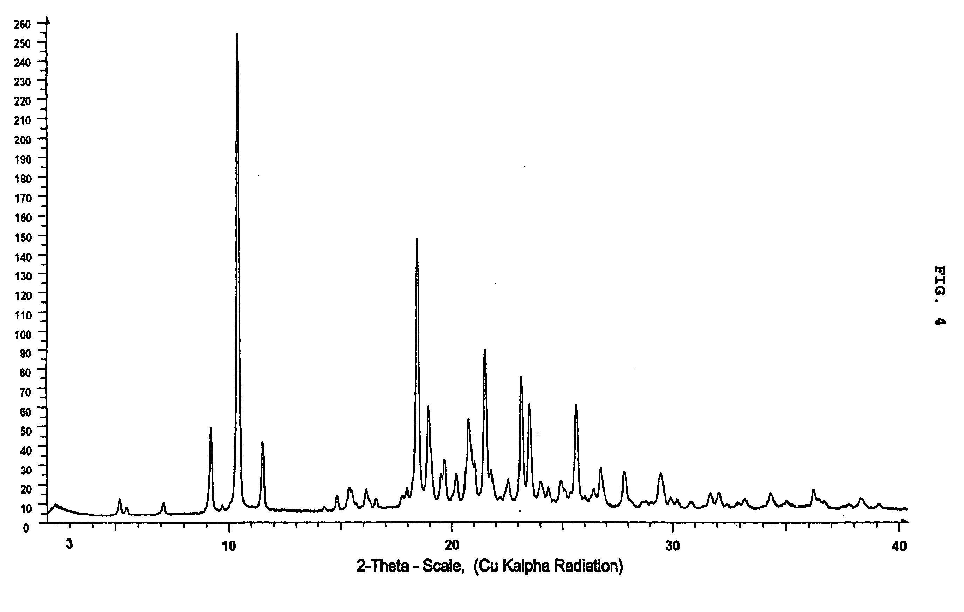

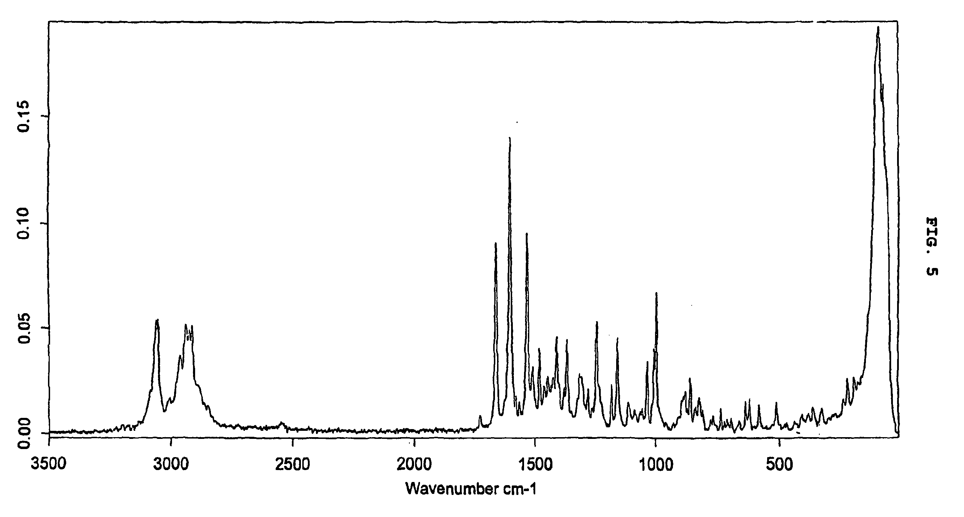

The obtained product is crystal A which is characterized by an X-ray powder diffraction pattern as shown in Figure 4 and further characterized by Raman spectroscopy giving the spectrum shown in Figure 5. Differential Scanning Calorimetry showed the sample to have a melting endotherm with a peak maximum at 98-100°C as given in Figure 6.

### Example 10

### Preparation of atorvastatin 4-(nitrooxy) butyl ester form A

80.0 mg of atorvastatin 4-(nitrooxy) butyl ester form B were dissolved in 5 ml of terbutyl methyl ether. 9 ml of heptane were added. The suspension was shaken for 1.5 h at room temperature, then further 0.4 ml of heptane were added. After further 16.5 h of shaking the solid was isolated and analysed. X-Ray powder diffraction pattern and Raman spectrum agree with that of form A given in Example 9.

### Stability Evaluation:

Stability at 40°C and 75% of relative humidity of atorvastatin 4-(nitrooxy) butyl ester A and form B in comparison with the amorphous form was assessed.
Atorvastatin 4-(nitrooxy) butyl ester form A, form B and amorphous were stored in open vials for one month at 40°C and in presence of 75% of relative humidity, and the samples were monitored by Raman spectroscopy and by PXRD after one and four weeks. The results show that while form A and form B showed no differences in the solid form after one and four weeks, PXRD and Raman analysis of amorphous atorvastatin 4-(nitrooxy) butyl ester indicated that the sample converts to form B. The results are summarized in the following Table 5.

**TABLE 5: Stability of amorphous, A and B forms at 40°C and 75% of relative humidity.**

| **STARTING MATERIAL** | **CONDITIONS** | **RESULTING FORM** |
|---|---|---|
| Form A | Storage at 40°C and 75% r.h.; 28 d. | Form A |
| Form B | Storage at 40°C and 75% r.h.; 28 d. | Form B |
| Amorphous | Storage at 40°C and 75% r.h.; 7 days. | B + traces of amorphous |
| Amorphous | Storage at 40°C and 75% r.h.; 28 days. | B |

Form A and form B atorvastatin 4-(nitrooxy) butyl ester are stable in the reported conditions for at least four weeks. Amorphous form resulted not stable, showing rapid conversion to form B when exposed to heat and humidity.
Stability of amorphous atorvastatin 4-(nitrooxy) butyl ester in aqueous suspension was assessed.
Amorphous atorvastatin 4-(nitrooxy) butyl ester was suspended in water at room temperature while stirring and monitored by Raman spectroscopy after one, seven and twenty-eight days. After one day the Raman spectrum corresponded to pure amorphous form; after 7 days a change in the intensity at 1664 cm⁻¹ indicated the start of crystallization of form B; after 28 days the Raman spectrum confirmed the crystallization in progress and further PXRD analysis showed a mixture of amorphous form and form B. The results are summarized in the following Table 6.

**TABLE 6: Stability of amorphous form in aqueous suspension**

| **STARTING MATERIAL** | **CONDITIONS** | **RESULTING FORM** |
|---|---|---|
| Amorphous | Suspension in H₂O; rt; 1 d. | Amorphous |
| Amorphous | Suspension in H₂O; rt; 7 d. | Amorphous (+B) |
| Amorphous | Suspension in H₂O; rt; 28 d. | Amorphous + B |

Amorphous atorvastatin 4-(nitrooxy) butyl ester resulted to be not stable in aqueous suspension at room temperature. After 7 days a detectable amount of form B was observed, and the further conversion was confirmed after following three weeks.

## Claims

1. A crystalline form B of (βR, δR)-2(4-fluorophenyl)- β, δ-dihydroxy-5-(1-methylethyl)-3-phenyl-4-[(phenylamino) carbonyl]-1H-pyrrole-1-heptanoic acid 4-(nitrooxy) butyl ester.

2. The crystalline form B according to claim 1, wherein the crystalline form is **characterized by** an X-ray powder diffraction pattern having peaks at 2-theta (2θ)= 9.47±0.1; 14.26±0.1; 15.03±0.1; 16.97±0.1; 18.70±0.1; 19.04±0.1; 19.71±0.1; 19.92±0.1; 20.82±0.1; 21.21+0.1; 21.77+0.1; 22.17±0.1; 22.44±0.1; 22.67±0.1; 23.97+0.1; 24.89±0.1; 25.24±0.1; 28.23±0.1; 30.36±0.1; 33.54±0.1.

3. The crystalline form B according to claim 1, wherein the crystalline form is **characterized by** an X-ray powder diffraction pattern as shown in Figure 1.

4. The crystalline form B according to any of claims 1 to 3, wherein the crystalline form is **characterized by** a Raman spectrum having main absorption peaks at wavelength (λ) cm⁻¹: 3064; 2963; 2947; 2943; 2918; 2890; 1665; 1603; 1560; 1528; 1508; 1481; 1452; 1435; 1409; 1400; 1365; 1311; 1241; 1182; 1159; 1036; 1006; 996; 825; 198; 112; 86.

5. A process for the preparation of the crystalline form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
stirring a suspension of amorphous (βR, δR)-2(4-fluorophenyl)- β, δ-dihydroxy-5-(1-methylethyl)-3-phenyl-4- [(phenylamino) carbon-yl]-1H-pyrrole-1-heptanoic acid 4-(nitrooxy) butyl ester in cumene at -5°C;
adding further cumene to complete the precipitation;
collecting the solid by filtration.

6. A process for the preparation of the crystalline form B of (βR, δR)-2(4-fluorophenyl)-β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
stirring a suspension of amorphous (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester in 1-octanol at 40°C;
adding further 1-octanol to complete the precipitation;
collecting the solid by filtration.

7. A process for the preparation of the crystalline form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
stirring a suspension of amorphous (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester in a mixture ethyl acetate/heptane 1:2 (V/V) at 5°C;
adding further cold ethyl acetate/heptane 1:2 (V/V) to complete the precipitation;
collecting the solid by filtration.

8. A process for the preparation of the crystalline form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
stirring a suspension of amorphous (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester in a mixture ethyl acetate/hexane 1:1 (V/V) at room temperature;
adding further hexane to complete the precipitation;
collecting the solid by filtration.

9. A process for the preparation of the crystalline form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
stirring a suspension of amorphous (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester in a mixture of toluene/ isopropyl ether about 1:2 (V/V) at 20-35°C;
collecting the solid by centrifugation.

10. A process for the preparation of the crystalline form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
dissolving amorphous (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester in a mixture of ethyl acetate/hexane 0.5:1 (V/V) by heating to 50°C; precipitating the solid by rapidly cooling the solution to 0°C; collecting the solid by filtration at room temperature.

11. A process for the preparation of the crystalline Form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
dissolving amorphous (βR, δR)-2(4-fluorophenyl)- β, 5-dihydroxy-5-(1-methylethyl)-3-phenyl-4-[(phenylamino) carbon-yl]-1H-pyrrole-1-heptanoic acid 4-(nitrooxy) butyl ester in a mixture of ethyl acetate/hexane 1:1 (V/V) at room temperature; precipitating the solid by exposing the solution to an hexane saturated atmosphere; collecting the solid by filtration.

12. A process for the preparation of the crystalline form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 1 to 4 comprising the following steps:
dissolving amorphous (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester in a mixture of ethyl acetate/hexane 2:1 (V/V) at room temperature; precipitating the solid by submitting the solution to an hexane saturated atmosphere;
collecting the solid by filtration.

13. Crystalline form B of (βR, δR)-2(4-fluorophenyl)- β, δ-dihydroxy-5-(1-methylethyl)-3-phenyl-4-[(phenylamino) carbonyl]-1H-pyrrole-1-heptanoic acid 4-(nitrooxy) butyl ester as defined in any of claims 1 to 4 for use as a medicament.

14. Compound as defined in any of claims 1 to 4 for use in the treatment of inflammation, thrombotic diseases and platelet aggregation activity.

15. Compound as defined in any of claims 1 to 4 for use in the treatment of acute coronary syndromes, stroke, peripheral vascular diseases and disorders associated with endothelial dysfunctions.

16. Compound as defined in any of claims 1 to 4 for use in the treatment of neurodegenerative and autoimmune disorders.

17. Compound as defined in any of claims 1 to 4 for use in the treatment of Alzheimer's disease, Parkinson's disease and multiple sclerosis.

18. A pharmaceutical composition comprising a pharmaceutically effective amount of the crystalline form B of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester as defined in any of claims 1 to 4 and a pharmaceutically acceptable adiuvant and/or carrier.

19. A pharmaceutical composition according to claim 18 in a suitable form for the oral, parenteral, rectal, and transdermic administration, by inhalation spray or aerosol.

20. A crystalline form A of (βR, δR)-2(4-fluorophenyl)- β, δ-dihydroxy-5-(1-methylethyl)-3-phenyl-4-[(phenylamino) carbonyl]-1H-pyrrole-1-heptanoic acid 4-(nitrooxy) butyl ester.

21. The crystalline form A according to claim 20, wherein the crystalline form is **characterized by** an X-ray powder diffraction pattern having peaks at 2-theta (2θ)= 9.19±0.1; 10.42±0.1; 11.49±0.1; 18.48±0.1; 18.98±0.1; 19.53±0.1; 19.68±0.1; 20.22±0.1; 20.80±0.1; 21.04±0.1; 21.52+0.1; 21.77±0.1; 23.16±0.1; 23.53±0.1; 25.66±0.1; 26.78±0.1; 27.85±0.1.

22. The crystalline form A according to claim 20, wherein the crystalline form is **characterized by** an X-ray powder diffraction pattern as shown in Figure 4.

23. The crystalline form A according to any of claims 20 to 22, wherein the crystalline form is **characterized by** a Raman spectrum having main absorption peaks at wavelength (λ) cm⁻¹: 3057; 2968; 2944; 2929; 2919; 1662; 1605; 1533; 1509; 1481; 1462; 1446; 1423; 1409; 1380; 1367; 1313; 1280; 1243; 1180; 1156; 1035; 1006; 997; 880; 857; 227; 201; 101; 85.

24. A process for the preparation of the crystalline form A of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 20 to 23 comprising the following steps:
dissolving (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester form B in terbutyl methyl ether at room temperature;
adding heptane;
shaking the suspension;
further adding heptane to complete the precipitation;
collecting the solid by filtration.

25. A process for the preparation of the crystalline form A of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester according to any of claims 20 to 23 comprising the following steps:
dissolving amorphous (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester in a mixture of ethyl acetate/hexane 2:1 1 (V/V) at room temperature; precipitating the solid by adding hexane;
shaking the suspension;
collecting the solid by filtration.

26. Crystalline form A of (βR, δR)-2(4-fluorophenyl)- β, δ-dihydroxy-5-(1-methylethyl)-3-phenyl-4-[(phenylamino) carbonyl]-1H-pyrrole-1-heptanoic acid 4-(nitrooxy) butyl ester form A as defined in any of claims 5 to 8 for use as a medicament.

27. Compound as defined in any of claims 20 to 23 for use in the treatment of inflammation, thrombotic diseases and platelet aggregation activity.

28. Compound as defined in any of claims 20 to 23 for use in the treatment of acute coronary syndromes, stroke, peripheral vascular diseases and all disorders associated with endothelial dysfunctions.

29. Compound as defined in any of claims 20 to 23 for use in the treatment of neurodegenerative and autoimmune disorders.

30. Compound as defined in any of claims 20 to 23 for use in the treatment of Alzheimer's disease, Parkinson's disease and multiple sclerosis.

31. A pharmaceutical composition comprising a pharmaceutically effective amount of the crystalline form A of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester as defined in any of claims 20 to 23 and a pharmaceutically acceptable adiuvant and/or carrier.

32. A pharmaceutical composition according to claim 31 in a suitable form for the oral, parenteral, rectal, topic and transdermic administration, by inhalation spray or aerosol.

33. A composition comprising crystalline form B or A of (βR, δR)-2(4-fluorophenyl)- β, δ-dihydroxy-5-(1-methylethyl)-3-phenyl-4-[(phenylamino) carbon-yl]-1H-pyrrole-1-heptanoic acid 4-(nitrooxy) butyl ester as defined in any of claims 1 to 4 or in any of claims 20 to 23 in combination with at least a compound used to treat cardiovascular diseases.

34. A composition according to claim 33, wherein the compound used to treat cardiovascular disease is selected from the group comprising: ACE inhibitors, angiotensin II receptor antagonists, beta-adrenergic blockers, calcium channel blockers, antithrombotics, aspirin, nitrosated ACE inhibitors, nitrosated angiotensin II receptor antagonists, nitrosated beta-adrenergic blockers and nitrosated aspirin.

## Patentansprüche

1. Kristalline Form B von (βR, δR) -2 (4-Fluorphenyl)-β,δ-dihydroxy-5-(1-methylethyl)-3-phenyl-4-[(phenylamino)carbonyl]-1H-pyrrol-1-hepansäure4-(nitrooxy)butylester.

2. Kristalline Form B gemäß Anspruch 1, worin die kristalline Form **gekennzeichnet ist durch** ein Röntgenstruktur Pulverdiffraktionsmuster mit Peaks bei 2-theta (2θ) - 9.47±0.1 ; 14.26±0.1; 15.03±0.1; 16.97±0.1; 18.70±0.1; 19.04±0.1; 19.7±0.1; 19.92±0.1; 20.82±0.1; 21.2±0.1; 21.77±0.1; 22.17±0.1; 22.44±0.1; 22.67±0.1; 23.97±0.1; 24.89±0.1; 25.24±0.1; 28.23±0.1: 30.36±0.1; 33.54±0.1.

3. Kristalline Form B gemäß Anspruch 1. worin die kristalline Form **gekennzeichnet ist durch** ein Röntgenstruktur Pulverdiffraktionsmuster wie in Figur 1 gezeigt.

4. Kristalline Form B gemäß einem der Ansprüche 1 bis 3 worin die kristalline Form charakterisiert ist durch ein Raman Spektrum mit Hauptabsorptionspeaks bei Wellenlängen (λ) cm⁻¹: 3064; 2963; 2947; 2943; 2918; 2890; 1665; 1603, 1560; 1528; 1508; 1481; 1452; 1435; 1409; 1400; 1365; 1311; 1241; 1182; 1159; 1036; 1006; 996; 825; 198; 112; 86.

5. Verfahren zur Herstellung der kristallinen Form B von (βR, δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrrooxy) butylester gemäß einem der Ansprüche 1 bis 4 umfassend die folgenden Schritte:
Rühren einer Suspension von amorphem (βR, δR)-2 (4-Fluorphenyl)-β,δ-dihydroxy-5-(1-methylethyl)-3-phenyl-4-[(phenylamino)carbonyl]-1 H-pyrrol-1-heptansäure 4-(nitrrooxy) butylester in Cumol bei - 5°C;
Hinzufügen weiteren Cumols um die Fällung zu vervollständigen;
Sammeln des Feststoffes durch Filtration.

6. Verfahren zur Herstellung der kristallinen Form B von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester gemäß einem der Ansprüche 1 bis 4 umfassend die folgenden Schritte:
Rühren einer Suspension von amorphem (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester in 1-Octanol bei 40°C;
Hinzufügen von weiterem 1-Octanol um die Fällung zu vervollständigen;
Sammeln des Feststoffes durch Filtration.

7. Vefahren zur Herstellung der kristallinen Form B von (βR,δR)-2 (4-Fluorphenyl)-β,δ-dihydroxy-5-(1-methylethyl)-3-phenyl-4-[(phenylamino]carbonyl]-1H-pyrrol-1-heptansäure 4-(nitrooxy) butylester gemäß einen der Ansprüche 1 bis 4 umfassend die folgenden Schritte:
Rühren einer Suspension von amorphem (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester in einer Mischung von Ethylacetat/Heptan 1:2 (V/V) bei 5°C;
Hinzufügen von weiterem kalten Ethylacetat/Heptan 1:2 (V/V) um die Fällung zu vervollständigen;
Sammeln des Feststoffes durch Filtration.

8. Verfahren zur Herstellung der kristallinen Form B von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester gemäß einen der Ansprüche bis 4 umfassend die folgenden Schritte:
Rühren einer Suspension von amorphem (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester in einer Mischung von Ethylactetat/Hexan 1:1 (V/V) bei Raumtemperatur;
Hinzufügen von weiterem Hexan um die Fällung zu vervollständigen;
Sammeln des Feststoffes durch Filtration.

9. Verfahren zur Herstellung der kristallinen Form B von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester gemäß einem der Ansprüche 1 bis 4 umfassend die folgenden Schritte:
Rühren einer Suspension von amorphem von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester in einer Mischung von Toluol/Isopropylether von etwa 1:2 (V/V) bei 20-35°C;
Sammeln des Feststoffes durch Zentrifugation.

10. Verfahren zur Herstellung der kristallinen Form B von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester gemäß einen der Ansprüche 1 bis 4 umfassend die folgenden Schritte:
Auflösen von amorphem (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester in einer Mischung von Ethylacetat/Hexan 0.5:1 (V/V) durch Erhitzen auf 50°C;
Ausfällen des Feststoffes durch schnelles Kühlen der Lösung auf 0°C;
Sammeln des Feststoffes durch Filtration bei Raumtemperatur.

11. Verfahren zu Herstellung der kristallinen Form B von (βR,δR)-2 (4-Fluorphenyl)-β,δ-dihydroxy-5-(1-methylethyl)-3-phenyl-4-[(phenylamino)carbonyl]-1 H-pyrrol-1-heptansäure 4-(nitrooxy) butylester gemäß einem der Ansprüche 1 bis 4 umfassend die folgenden Schritte:
Auflösen von (βR,δR)-2 (4-Fluorphenyl)-β,δ-dihydroxy-5-(1-methylethyl)-3-phenyl-4-[(phenylamino)carbonyl]-1 H-pyrrol-1-heptansäure A-(nitrooxy) butylester in einer Mischung von Ethylacetat/Hexan 1:1 (V/V) bei Raumtemperatur;
Ausfällen des Feststoffes durch Aussetzen der Lösung einer Hexan gesättigten Atmosphäre;
Sammeln des Feststoffes durch Filtration.

12. Verfahren zur Herstellung der kristallinen Form B (βR,δR)-2 (4-Fluorphenyl)-β,δ-dihydroxy-5-(1-methylethyl)-3-phenyl-4-([phenylamino)carbonyl]-1H-pyrrol-1-heptansäure 4-(nitrooxy) butylester gemäß einem der Ansprüche 1 bis 4 umfassend die folgenden Schritte:
Auflösen von amorphem (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester in einer Mischung von Ethylacetat/Hexan 2:1 (V/V) bei Raumtemperatur;
Ausfällen des Feststoffes durch Aussetzen der Lösung einer Hexan gesättigten Atmosphäre;
Sammeln des Feststoffes durch Filtration.

13. Kristalline Form B von (βR,δR)-2 (4-Fluorphenyl)-β,δ-dihydroxy-5-(1-methylethyl)-3-phenyl-4-[(phenylamino)carbonyl]-1 H-pyrrol-1-heptansäure 4-(nitrooxy) butylester wie in einem der Ansprüche 1 bis 4 definiert zur Verwendung als Arzneimittel.

14. Verbindung wie in einem der Ansprüche 1 bis 4 definiert zur Verwendung in der Behandlung von Entzündungen, thrombotischer Erkrankungen und Blutplättchenaggregationsaktivität.

15. Verbindung wie in einem der Ansprüche 1 bis 4 definiert zur Verwendung in der Behandlung von akuten koronaren Syndromen, Schlaganfall, peripheren vaskulären Erkrankungen und Störungen, die mit endothelen Dysfunktionen assoziiert sind.

16. Verbindung wie in einem der Ansprüche 1 bis 4 definiert zur Verwendung in der Behandlung von neurodegenerativen und Autoimmunerkrankungen.

17. Verbindung wie in einem der Ansprüche 1 bis 4 definiert zur Verwendung in der Behandlung von Alzheimererkrankungen, Parkinsonerkrankungen und Multipler Sklerose.

18. Pharmazeutische Zusammensetzung enthalten einen pharmazeutisch wirksamen Gehalt der kristallinen Form B von (βR,δR)-2 (4-Fluorphenyl)-β,δ-dihydroxy-5-(1-methylethyl)-3-phenyl-4-[(phenylamino)carbonyl]-1 H-pyrrol-1-heptansäure 4-(nitrooxy) butylester wie in einem der Ansprüche 1 bis 4 definiert und einen pharmazeutisch verträglichen Hilfsstoff und/- oder Trägerstoff.

19. Pharmazeutische Zusammensetzung gemäß Anspruch 18 in geeigneter Form für die orale, parenterale, rektale und transdermale Verabreichung, als Inhalationsspray oder Aerosol.

20. Kristalline Form A von (βR,δR)-2 (4-Fluorphenyl)-β,δ-dihydroxy-5-(1-methylethyl)-3-phenyl-4-[(phenylamino)carbonyl]-1 H-pyrrol-1-heptansäure 4-(nitrooxy) butylester.

21. Kristalline Form A gemäß Anspruch 20, worin die kristalline Form charakterisiert ist, durch ein Röntgenstruktur Pulverdiffraktionsmuster mit Peaks bei 2-theta (2θ) - 9.19±0.1; 10.42±0.1, 11.49±0.1 ; 18.48±0.1, 18.98±0.1, 19.53±0.1 ; 19.68±0.1; 20.22±0.1; 20.80±0.1; 21.04±0.1; 21.52±0.1; 21.77±0.1; 23.16±0.1; 23.53±0.1; 25.66±0.1; 26.78±0.1; 27.85±0.1.

22. Kristalline Form A gemäß Anspruch 20, worin die kristalline Form charakterisiert ist durch ein Röntgenstruktur Pulverdiffraktionsmuster wie in Figur 4 gezeigt.

23. Kristalline Form A gemäß einem der Ansprüche 20 bis 22, worin die kristalline Form charakterisiert ist durch ein Raman Spektrum, mit Hauptabsorptionspeaks bei Wellenlängen (λ) cm⁻¹: 3057; 2968; 2944; 2929; 2919; 1662; 1605; 1533; 1509; 1481; 1462; 1446; 1423; 1409; 1380; 1367; 1313; 1280; 1243; 1180; 1156; 1035; 1006; 997; 880; 857; 227; 201; 101; 85.

24. Verfahren zur Herstellung der kristallinen Form A von (βR,δR)-2 {4-Fluorphenyl]-β,δ-dihydroxy-5-(1-methylethyl)-3-phenyl-4-[(phenylamino)carbonyl]-1 H-pyrrol-1-heptansäure 4-(nitrooxy) butylester gemäß einen der Ansprüche 20 bis 23 umfassend die folgenden Schritte:
Auflösen von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester Form B in Tertbutylmethylether bei Raumtemperatur;
Hinzufügen von Heptan;
Schütteln der Suspension;
Hinzufügen von weiterem Heptan um die Fällung zu vervollständigen;
Sammeln des Feststoffes durch Filtration.

25. Verfahren der Erstellung der kristallinen Form A (βR,δR)-2 (4-Fluorphenyl)-β,δ-dihydroxy-5-(1-methylethyl)-3-phenyl-4-[(phenylamino)carbonyl]-1 H-pyrrol-1-heptansäure 4-(nitrooxy) butylester gemäß einen der Ansprüche 20 bis 23 umfassend die folgenden Schritte:
Auflösen von amorphem (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester in einer Mischung von Ethylacetat/Hexan 2:1 (V/V) bei Raumtemperatur;
Ausfällen des Feststoffes durch hinzufügen von Hexan;
Schütteln der Suspension;
Sammeln des Feststoffes durch Filtration.

26. Kristalline Form A von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester Form A wie in einem der Ansprüche 5 bis 8 definiert zur Verwendung als Arzneimittel.

27. Verbindung wie in einem der Ansprüche 20 bis 23 definiert zur Verwendung in der Behandlung von Entzündungen, thrombotischen Erkrankungen und Blutplättchenaggregationsaktivität .

28. Verbindung wie in einem der Ansprüche 20 bis 23 definiert zur Verwendung in der Behandlung von akuten koronaren Syndromen, Schlaganfall, peripheren vaskulären Erkrankungen und Störungen, die mit endothelen Dysfunktionen assoziiert sind.

29. Verbindung wie in einem der Ansprüche 20 bis 23 definiert zur Verwendung in der Behandlung von neurodedegenerativen und Autoimmunerkrankungen.

30. Verbindung wie in einem der Ansprüche 20 bis 23 definiert zur Verwendung in der Behandlung von Alzheimererkrankungen, Parkinsonerkrankungen und Multipler Sklerose.

31. Pharmazeutische Zusammensetzung enthaltend einen pharmazeutisch wirksamen Gehalt der kristallinen Form A von [βP,δR)-2 [4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester wie in einem der Ansprüche 20 bis 23 definiert und ein pharmazeutisch verträgliches Hilfsmittel und/- oder Trägermaterial.

32. Pharmazeutische Zusammensetzung gemäß Anspruch 31 in einer geeigneten Form für die orale, parenterale, rektale, topische und transdermale Verabreichung, als Inhalationsspray oder Aerosol.

33. Zusammensetzung enthaltend die kristalline Form B oder A von (βR,δR)-2 (4-Fluorphenyl)-β,δ-dihydroxy-5-(1-methylethyl)-3-phenyl-4-[(phenylamino)carbonyl]-1H-pyrrol-1-heptansäure 4-(nitrooxy) butylester wie in einem der Ansprüche 1 bis 4 oder in einem der Ansprüche 20 bis 23 definiert in Kombination mit mindestens einer Verbindung die zur Behandlung kardiovaskulärer Erkrankungen verwendet wird.

34. Zusammensetzung gemäß Anspruch 33, worin die Verbindung die zur Behandlung kardiovaskulärer Erkrankungen verwendet wird ausgewählt ist aus der Gruppe umfassend: ACE Inhibitoren, Angiotensin II Rezeptor Antagonisten, beta-adrenerge Blocker, Calciumkanalblocker, Antithrombosemittel, Aspirin, nitrosierte ACE Inhibitoren, nitrosierte Angiotensin II Rezeptor Antagonisten, nitrosierte beta-adrenerge Blocker und nitrosiertes Aspirin.

## Revendications

1. Forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque.

2. Forme cristalline B selon la revendication 1, dans laquelle la forme cristalline est **caractérisée par** un diagramme de diffraction des rayons X sur poudre ayant des pics 2-thêta (2θ) = 9,47 ± 0,1 ; 14,26 ± 0,1 ; 15,03 ± 0,1 ; 16,97 ± 0,1 ; 18,70 ± 0,1 ; 19,04 ± 0,1 ; 19,71 ± 0,1 ; 19,92 ± 0,1 ; 20,82 ± 0,1 ; 21,21 ± 0,1 ; 21,77 ± 0,1 ; 22,17 ± 0,1 ; 22,44 ± 0,1 ; 22,67 ± 0,1 ; 23,97 ± 0,1 ; 24,89 ± 0,1 ; 25,24 ± 0,1 ; 28,23 ± 0,1 ; 30,36 ± 0,1 ; 33,54 ± 0,1.

3. Forme cristalline B selon la revendication 1, dans laquelle la forme cristalline est **caractérisée par** un diagramme de diffraction des rayons X sur poudre tel que présenté sur la figure 1.

4. Forme cristalline B selon l'une quelconque des revendications 1 à 3, dans laquelle la forme cristalline est **caractérisée par** un spectre Raman ayant les principaux pics d'absorption à la longueur d'onde (λ) cm⁻¹ :
3064 ; 2963 ; 2947 ; 2943 ; 2918 ; 2890 ; 1665 ; 1603 ; 1560 ; 1528 ; 1508 ; 1481 ; 1452 ; 1435 ; 1409 ; 1400 ; 1365 ; 1311 ; 1241 ; 1182 ; 1159 ; 1036 ; 1006 ; 996 ; 825 ; 198 ; 112 ; 86.

5. Procédé pour la préparation de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 1 à 4, comprenant les étapes suivantes :
agitation d'une suspension d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque amorphe dans du cumène à -5°C ;
ajout de cumène supplémentaire pour achever la précipitation ;
recueil du solide par filtration.

6. Procédé pour la préparation de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 1 à 4, comprenant les étapes suivantes :
agitation d'une suspension d'ester 4-(nitro-oxy)butylique d'acide (βR,δR)-(4-fluorophényl)-β,δ-dihydroxy-5-(1-méthyléthyl)-3-phényl-4-[(phénylamino)carbon-yl]-1H-pyrrole-1-heptanoïque amorphe dans du 1-octanol à 40°C ;
ajout de 1-octanol supplémentaire pour achever la précipitation ;
recueil du solide par filtration.

7. Procédé pour la préparation de la forme cristalline B de l'ester 4-(nitro-oxy)butylique d'acide (βR,δR)-2(4-fluorophényl)-β,δ-dihydroxy-5-(1-méthyléthyl) - 3-phényl-4-[(phénylamino)carbon-yl]-1H-pyrrole-1-heptanoique selon l'une quelconque des revendications 1 à 4 comprenant les étapes suivantes :
agitation d'une suspension d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque amorphe dans un mélange d'acétate d'éthyle/heptane 1:2 (V/V) à 5°C ;
ajout d'acétate d'éthyle/heptane 1:2 (V/V) froid supplémentaire pour achever la précipitation ;
recueil du solide par filtration.

8. Procédé pour la préparation de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 1 à 4 comprenant les étapes suivantes :
agitation d'une suspension d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque amorphe dans un mélange d'acétate d'éthyle/hexane 1:1 (V/V) à la température ambiante ;
ajout d'hexane supplémentaire pour achever la précipitation ;
recueil du solide par filtration.

9. Procédé pour la préparation de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 1 à 4 comprenant les étapes suivantes :
agitation d'une suspension d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque amorphe dans un mélange de toluène/éther isopropyique environ 1:2 (V/V) à une température de 20 à 35°C ;
recueil du solide par filtration.

10. Procédé pour la préparation de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 1 à 4 comprenant les étapes suivantes :
dissolution d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque amorphe dans un mélange d'acétate d'éthyle/hexane 0,5:1 (V/V) par chauffage à 50°C ; précipitation du solide par refroidissement rapide de la solution à 0°C ; recueil du solide par filtration à la température ambiante.

11. Procédé pour la préparation de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 1 à 4 comprenant les étapes suivantes :
dissolution d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoique amorphe dans un mélange d'acétate d'éthyle/hexane 1:1 (V/V) à la température ambiante ; précipitation du solide par exposition de la solution à une atmosphère saturée d'hexane ; recueil du solide par filtration.

12. Procédé pour la préparation de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 1 à 4 comprenant les étapes suivantes :
dissolution d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque amorphe dans un mélange d'acétate d'éthyle/hexane 2:1 (V/V) à la température ambiante ; précipitation du solide par soumission de la solution à une atmosphère saturée d'hexane ;
recueil du solide par filtration.

13. Forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque telle que définie selon l'une quelconque des revendications 1 à 4 destinée à une utilisation en tant que médicament.

14. Composé tel que défini selon l'une quelconque des revendications 1 à 4, destiné à une utilisation dans le traitement de l'inflammation, des maladies thrombotiques et de l'activité d'agrégation plaquettaire.

15. Composé tel que défini selon l'une quelconque des revendications 1 à 4, destiné à une utilisation dans le traitement des syndromes coronariens aigus, d'un accident vasculaire cérébral, des maladies vasculaires périphériques et des troubles associés à des dysfonctionnements endothéliaux.

16. Composé tel que défini selon l'une quelconque des revendications 1 à 4, destiné à une utilisation dans le traitement des troubles neurodégénératifs et auto-immuns.

17. Composé tel que défini selon l'une quelconque des revendications 1 à 4, destiné à une utilisation dans le traitement de la maladie d'Alzheimer, la maladie de Parkinson et la sclérose en plaques.

18. Composition pharmaceutique comprenant une quantité pharmaceutiquement efficace de la forme cristalline B de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque telle que définie selon l'une quelconque des revendications 1 à 4 et un adjuvant et/ou un support pharmaceutiquement acceptable.

19. Composition pharmaceutique selon la revendication 18 sous une forme appropriée pour l'administration orale, parentérale, rectale, et transdermique, par pulvérisation pour inhalation ou aérosol.

20. Forme cristalline A de l'ester 4- (nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque.

21. Forme cristalline A selon la revendication 20, dans laquelle la forme cristalline est **caractérisée par** un diagramme de diffraction des rayons X sur poudre ayant des pics 2-thêta (2θ) = 9,19 ± 0,1 ; 10,42 ± 0,1 ; 11,49 ± 0,1 ; 18,48 ± 0,1 ; 18,98 ± 0,1 ; 19,53 ± 0,1 ; 19,68 ± 0,1 ; 20,22 ± 0,1 ; 20,80 ± 0,1 ; 21,04 ± 0,1 ; 21,52 ± 0,1 ; 21,77 ± 0,1 ; 23,16 ± 0,1 ; 23,53 ± 0,1 ; 25,66 ± 0,1 ; 26,78 ± 0,1 ; 27,85 ± 0,1.

22. Forme cristalline A selon la revendication 20, dans laquelle la forme cristalline est **caractérisée par** un diagramme de diffraction des rayons X sur poudre tel que présenté sur la figure 4.

23. Forme cristalline A selon l'une quelconque des revendications 20 à 22, dans laquelle la forme cristalline est **caractérisée par** un spectre Raman ayant les principaux pics d'absorption à la longueur d'onde (λ) cm⁻¹ :
3057 ; 2968 ; 2944 ; 2929 ; 2919 ; 1662 ; 1605 ; 1533 ; 1509 ; 1481 ; 1462 ; 1446 ; 1423 ; 1409 ; 1380 ; 1367 ; 1313 ; 1280 ; 1243 ; 1180 ; 1156 ; 1035 ; 1006 ; 997 ; 880 ; 857 ; 227 ; 201 ; 101 ; 85.

24. Procédé pour la préparation de la forme cristalline A de l'ester 4-(nitro-oxy)butylique d'acide (βR,δR)-2(4-fluorophényl)-β,δ-dihydroxy-5-(1-méthyléthyl)-3-phényl-4-[(phénylamino)carbonyl]-1H-pyrrole-1-heptanoique selon l'une quelconque des revendications 20 à 23 comprenant les étapes suivantes :
dissolution de la forme B de l'ester 4-(nitro-oxy) butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque dans du tert-butyle méthyl éther à la température ambiante ;
ajout d'heptane ;
agitation de la suspension ;
ajout supplémentaire d'heptane pour achever la précipitation ;
recueil du solide par filtration.

25. Procédé pour la préparation de la forme cristalline A de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque selon l'une quelconque des revendications 20 à 23, comprenant les étapes suivantes :
dissolution d'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque amorphe dans un mélange d'acétate d'éthyle/hexane 2:1 (V/V) à la température ambiante ; précipitation du solide par l'ajout d'hexane ;
agitation de la suspension ;
recueil du solide par filtration.

26. Forme cristalline A de la forme A de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque telle que définie selon l'une quelconque des revendications 5 à 8 destinée à une utilisation en tant que médicament.

27. Composé tel que défini selon l'une quelconque des revendications 20 à 23, destiné à une utilisation dans le traitement de l'inflammation, des maladies thrombotiques et de l'activité d'agrégation plaquettaire.

28. Composé tel que défini selon l'une quelconque des revendications 20 à 23, destiné à une utilisation dans le traitement des syndromes coronariens aigus, d'un accident vasculaire cérébral, des maladies vasculaires périphériques et de tous les troubles associés à des dysfonctionnements endothéliaux.

29. Composé tel que défini selon l'une quelconque des revendications 20 à 23, destiné à une utilisation dans le traitement des troubles neurodégénératifs et auto-immuns.

30. Composé tel que défini selon l'une quelconque des revendications 20 à 23, destiné à une utilisation dans le traitement de la maladie d'Alzheimer, la maladie de Parkinson et la sclérose en plaques.

31. Composition pharmaceutique comprenant une quantité pharmaceutiquement efficace de la forme cristalline A de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque telle que définie selon l'une quelconque des revendications 20 à 23 et un adjuvant et/ou un support pharmaceutiquement acceptable.

32. Composition pharmaceutique selon la revendication 31 sous une forme appropriée pour l'administration orale, parentérale, rectale, topique et transdermique, par pulvérisation pour inhalation ou aérosol.

33. Composition comprenant la forme cristalline B ou A de l'ester 4-(nitro-oxy)butylique d'acide (βR,δR)-2(4-fluorophényl)-β,δ-dihydroxy-5-(1-méthyléthyl)-3-phényl-4-[(phénylamino)carbon-yl]-1H-pyrrole-1-heptanoïque telle que définie selon l'une quelconque des revendications 1 à 4 ou selon l'une quelconque des revendications 20 à 23 en combinaison avec au moins un composé utilisé pour traiter les maladies cardio-vasculaires.

34. Composition selon la revendication 33, dans laquelle le composé utilisé pour traiter une maladie cardio-vasculaire est choisi dans le groupe comprenant :
les inhibiteurs de l'ECA, les antagonistes du récepteur de l'angiotensine II, les bêta-bloquants adrénergiques, les inhibiteurs calciques, les antithrombotiques, l'aspirine, les inhibiteurs de l'ECA nitrosés, les antagonistes du récepteur de l'angiotensine II nitrosés, les bêta-bloquants adrénergiques nitrosés et l'aspirine nitrosé.
